(19) **Europäisches Patentamt**
**European Patent Office**
**Office européen des brevets**

(11) **EP 2 474 962 A1**

(12) **EUROPÄISCHE PATENTANMELDUNG**

(43) Veröffentlichungstag:
**11.07.2012 Patentblatt 2012/28**

(51) Int Cl.:
**_G09B 23/00_** _(2006.01)_

(21) Anmeldenummer: **11000081.7**

(22) Anmeldetag: **07.01.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder:
• **Roche Diagnostics GmbH**
**68305 Mannheim (DE)**
Benannte Vertragsstaaten:
**DE**
• **F.Hoffmann-La Roche AG**
**4070 Basel (CH)**
Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(72) Erfinder:
• **Staib Arnulf**
**64646 Heppenheimd (DE)**
• **Quarder Ortrud**
**69115 Heidelberg (DE)**
• **Werner Gerhard**
**69469 Weinheim (DE)**

(74) Vertreter: **Mommer, Niels**
**Twelmeier Mommer & Partner**
**Westliche Karl-Friedrich-Strasse 56-68**
**75172 Pforzheim (DE)**

(54) **Verfahren zur Auswertung eines Satzes von Messdaten eines oralen Glucosetoleranztests**

(57) Die Erfindung betrifft ein Verfahren zur Auswertung eines Satzes von Messdaten eines oralen Glucosetoleranztests, wobei der Satz von Messdaten eine Folge von Messdaten der Glucosekonzentration und zusätzlich wenigstens eine Folge von Messwerten einer weiteren Analytkonzentration enthält. Erfindungsgemäß ist vorgesehen, dass aus der Folge von Messwerten der Glucosekonzentration und jeweils einem von mehreren vorgegebenen Glucose-Musterverläufen jeweils ein Wert eines Ähnlichkeitsmaß berechnet wird, das die Ähnlichkeit zwischen dem zeitlichen Verlauf der Folge von Messwerten der Glucosekonzentration und dem betreffenden Glucose-Musterverlauf quantifiziert, aus der Folge von Messwerten der weiteren Analytkonzentration und mehreren vorgegebenen Analyt-Musterverläufen jeweils ein Wert eines weiteren Ähnlichkeitsmaß berechnet wird, das die Ähnlichkeit zwischen dem Verlauf der Folge von Messwerten der weiteren Analytkonzentration und dem betreffenden Analyt-Musterverlauf quantifiziert, der Datensatz in einem Vektorraum, der von den Ähnlichkeitsmaßen gebildete Koordinatenachsen aufweist, durch einen Punkt charakterisiert wird, dessen Koordinaten die berechneten Werte der Ähnlichkeitsmaße enthalten, die Lage dieses Punkts in Bezug auf Referenzpunkte, die jeweils einen definierten Gesundheitszustand repräsentieren, ausgewertet wird, um einen Parameter zu berechnen, der den Zustand des Glucosestoffinrechsels des Patienten angibt.

Fig. 6

**Beschreibung**

Die Erfindung betrifft ein Verfahren zur Auswertung eines Satzes von Messdaten eines oralen Glucosetoleranztests.

Bei einem oralen Glucosetoleranztest wird einem Patienten in nüchternem Zustand oral Glucoselösung verabreicht und anschließend in zeitlichen Abständen die Glucosekonzentration im Blut des Patienten gemessen. In der Regel wird der Verlauf der Blutzuckerkonzentration über einen Zeitraum von 2 Stunden aufgezeichnet. Ein Glucosetoleranztest kann aber auch über einen kürzeren oder längeren Zeitraum durchgeführt werden.

Indem der Verlauf der Glucosekonzentration als Reaktion auf die Einnahme von Glucose ermittelt wird, können Auffälligkeiten des Glucosemetabolismus erkannt werden. Orale Glucosetoleranztests ermöglichen deshalb den Nachweis einer gestörten Glucoseverwertung und die Diagnose von Diabetes.

Ein Verfahren zur Auswertung eines Satzes von Messdaten eines oralen Glucosetoleranztests ist aus der EP 1 830 333 A1 bekannt. Bei dem bekannten Verfahren wird der menschliche Glucosemetabolismus durch ein System von Differentialgleichungen beschrieben, die verschiedene freie Parameter enthalten. Diese Parameter werden numerisch so angepasst, dass das Modell den gemessenen Verlauf der Glucosekonzentration möglichst gut wiedergibt. Der Wert der auf diese Weise bestimmten Parameter ist für verschiedene Gesundheitszustände, d. h. Stadien einer Diabeteserkrankung, charakteristisch, so dass durch Auswertung der Parameterwerte der Gesundheitszustand eines Patienten bestimmt werden kann.

Ein Nachteil bei dem bekannten Verfahren ist, dass die Interpretation der gewonnenen Ergebnisse einige Erfahrung und vertiefte mathematische Kenntnis erfordert. Insbesondere ist es schwierig, numerische Worte für die Parameter des Differentialgleichungssystems den Krankheitszuständen zuzuordnen. Zudem lassen sich frühe Stadien einer Diabeteserkrankung und prädiabetische Zustände, die noch keine externe Insulinzufuhr und häufig auch noch keine medikamentöse Behandlung erforderlich machen, anhand der Parametersätze des Differentialgleichungssystems nur unzureichend erkennen und voneinander unterscheiden. Von großem Interesse ist jedoch nicht nur eine manifeste Diabeteserkrankung zu erkennen, sondern auch bereits eine sich abzeichnende Erkrankung oder ein erhöhtes Krankheitsrisiko feststellen zu können.

Aufgabe der vorliegenden Erfindung ist es, einen Weg aufzuzeigen, wie die Auswertung von Messdaten oraler Glucosetoleranztests verbessert werden kann. Insbesondere soll das Ergebnis der Auswertung ohne vertiefte mathematische Kenntnisse verständlich sein und es ermöglichen, prädiabetische Gesundheitszustände zu erkennen.

Diese Aufgabe wird durch ein Verfahren mit den im Anspruch 1 angegebenen Merkmalen gelöst. Vorteilhafte Weiterbildungen der Erfindung sind Gegenstand von Unteransprüchen.

Die Aussagekraft eines oralen Glucosetoleranztests lässt sich verbessern, indem zusätzlich zu dem Verlauf der Glucosekonzentration auch der Verlauf von weiteren Analyten in einer Körperflüssigkeit des Patienten, in der Regel Blut und/oder interstitieller Flüssigkeit, gemessen wird. Geeignet sind insbesondere Sekretionshormone wie Insulin, Proinsulin, Glukagon oder C-Peptid. Bei einem erfindungsgemäßen Verfahren wird deshalb ein Satz von Messdaten eines oralen Glucosetoleranztests ausgewertet, der eine Folge von Messdaten der Glucosekonzentration und zusätzlich wenigstens eine Folge von Messdaten einer weiteren Analytkonzentration enthält, ausgewertet.

Zunächst wird dazu aus der Folge von Messwerten der Glucosekonzentration und jeweils einem von mehreren vorgegebenen Glucose-Musterverläufen jeweils ein Wert eines Ähnlichkeitsmaßes berechnet, das die Ähnlichkeit zwischen dem zeitlichen Verlauf der Folge von Messwerten der Glucosekonzentration und dem betreffenden Glucose-Musterverlauf quantifiziert.

Als Glucose-Musterverlauf kann beispielsweise die für einen Patienten mit einem bestimmten Gesundheitszustand bei einem Glucosetoleranztest zu erwartende Verlaufskurve der Glucosekonzentration verwendet werden. Entsprechende Musterverläufe können durch Untersuchung von Musterpatienten, für die bereits auf anderem Wege eine Diagnose gestellt wurde, ermittelt werden, insbesondere für einen völlig gesunden Gesundheitszustand (H-NG), einen Patienten mit Typ-II-Diabetes (DT2), einem normoglykämischen Musterpatienten mit metabolischem Syndrom (MS-NG), einem Musterpatienten mit metabolischem Syndrom und gestörter Glucosetoleranz (MS-IGT), einem Musterpatienten mit metabolischem Syndrom und gestörter Nüchternglucose (MS-IFG) und einem Musterpatienten, bei dem die Erhöhung der Nüchternglucose mit gestörter Glucosetoleranz (MS-CGI) kombiniert ist. Allerdings sind derartige Verlaufskurven mathematisch aufwändig zu beschreiben und machen deshalb einen hohen Rechenaufwand bei der Auswertung erforderlich.

Es ist deshalb vorteilhaft, Glucose-Musterverläufe zu verwenden, beispielsweise Funktionen, die zumindest abschnittsweise linear in der Zeit sind. Schon mit derartig einfachen Funktionen lassen sich Abschnitte des zeitlichen Verlaufs der Glucosekonzentration bei einem Gesundheitszustand charakterisieren. Insbesondere können lineare oder abschnittsweise lineare Funktionen verwendet werden, die lediglich nährungsweise den Anstieg oder Abfall der Glucosekonzentration während eines Teils der Zeitdauer eines Glucosetoleranztests für einen bestimmten Gesundheitszustand beschreiben.

Zwingend erforderlich ist eine Ähnlichkeit der Musterverläufe zu einem tatsächlichen Verlauf aber nicht, da beispielsweise auch ein Satz von hinreichend unterschiedlichen Funktionen verwendet werden kann, aus denen beispielsweise durch lineare Kombination reale Verlaufsprofile angenährt werden können, beispielsweise als Fourierreihe. Ein geeigneter Satz von Funktionen kann beispielsweise aus Polynomen bestehen, insbesondere Polynomen der Form $x^n$.

Aus der Folge von Messwerten der Glucosekonzentration und einem Glucose-Musterverlauf wird erfindungsgemäß ein Wert eines Ähnlichkeitsmaßes berechnet. Als Ähnlichkeitsmaß ist an sich jede Berechnungsvorschrift geeignet, die ein umso höheres Ergebnis liefert, je größer die Übereinstimmung zwischen der Serie von Messdaten der Glucosekonzentration und dem betrachteten Musterverlauf ist.

Beispielsweise kann als Ähnlichkeitsmaß ein Korrelationskoeffizient verwendet werden, insbesondere ein Pearsonscher Maßkorrelationskoeffizient. Bevorzugt wird das Ähnlichkeitsmaß aber als Skalarprodukt von zwei Vektoren berechnet, wobei einer der beiden Vektoren aus der betreffenden Folge von Messwerten und der andere Vektor aus dem betreffenden Musterverlauf bestimmt wird. Beispielsweise können die einzelnen Messwerte $g_1$, $g_2$, $g_3$ bis $g_n$, die jeweils zu aufeinanderfolgenden Zeitpunkten $t_1$, $t_2$ bis $t_n$ gemessen wurden, als Komponenten eines Vektors verwendet werden. In entsprechender Weise kann aus einem Musterverlauf ebenfalls ein Vektor gebildet werden, indem der Konzentrationswert des Musterverlaufs bei dem entsprechenden Zeitpunkt $t_1$, $t_2$ bis $t_n$ als Vektorkomponente verwendet wird.

Besonders vorteilhaft ist es dabei, die Ähnlichkeitsmaße aus normierten Konzentrationsverläufen zu berechnen, beispielsweise als Skalarprodukt von normierten Vektoren zu berechnen. Auf diese Weise lässt sich der mathematische Aufwand vereinfachen und der deskriptive Wert des Ähnlichkeitsmaßes erhöhen, da nur der relative Verlauf der Glucosekonzentration und somit die Form des Verlaufsprofils betrachtet wird.

In entsprechender Weise wird erfindungsgemäß aus der Folge von Messwerten der weiteren Analytkonzentration und mehreren vorgegebenen Analyt-Musterverläufen jeweils ein Wert eines weiteren Ähnlichkeitsmaß berechnet, das die Ähnlichkeit zwischen dem Verlauf der Folge von Messwerten der weiteren Analytkonzentration und dem betreffenden Analyt-Musterverlauf quantifiziert. Als Analyt-Musterverläufe können die für einen Patienten mit einem bestimmten Gesundheitszustand bei einem Glucosetoleranztest zu erwartende Verlaufskurve der Analytkonzentration verwendet werden. Ebenso wie im Fall der Glucose-Musterverläufe ist es aber auch bei Anyalyt-Musterverläufen vorteilhaft, Funktionen zu verwenden, die zumindest abschnittsweise linear sind.

Wichtig ist dabei, dass das Ähnlichkeitsmaß im Bezug auf den Verlauf der Glucosekonzentration und der Verlauf der weiteren Analytkonzentration nicht unbedingt für dieselben bzw. alle zur Verfügung stehenden Gesundheitszustände berechnet werden. Es zeigen sich nämlich nicht für jeden Analyten signifikante Unterschiede im Verlauf der Analytkonzentration zwischen allen Gesundheitszuständen. Prädiabetische Gesundheitszustände und beginnende Stadien einer Diabeteserkrankung können in weiten Teilen des Analyt- oder Glucose-Konzentrationsverlaufs übereinstimmen, so dass für die betreffende Abschnitte des Konzentrationsverlaufs und die betreffenden Gesundheitszustände auf die Berechnung eines Ähnlichkeitsmaßes verzichtet werden kann, ohne die Aussagekraft der Aushärtung zu verschlechtern.

Erfindungsgemäß wird aus dem Satz von Messdaten eines oralen Glucosetoleranztests unter Verwendung verschiedener Musterverläufe also zunächst ein Satz von Ähnlichkeitsmaßen berechnet, also ein Satz von Variablen, die jeweils ein Maß für die Ähnlichkeit zwischen dem Verlauf der Folge von Messwerten und einem Musterverlauf sind.

In einem weiteren Schritt des erfindungsgemäßen Verfahrens wird ein Vektorraum betrachtet, der von den Ähnlichkeitsmaßen gebildete Koordinatenachsen aufweist. In diesem Vektorraum wird der Datensatz eines Individuums mit unbekanntem Krankheitszustand durch einen Punkt charakterisiert, dessen Koordinaten die berechneten Werte der Ähnlichkeitsmaße enthalten. Wenn also beispielsweise eine erste Koordinatenachse des Vektorraums von dem Ähnlichkeitsmaß zwischen dem zeitlichen Verlauf der Folge von Messwerten der Glucosekonzentration und einem ersten Glucose-Musterverlauf gebildet ist, stellt der Wert dieses Ähnlichkeitsmaßes die erste Koordinate des den Datensatz repräsentierenden Punkts dar.

Bei einem erfindungsgemäßen Verfahren wird die Lage dieses Punkts in Bezug auf Referenzpunkte, die jeweils einen definierten Gesundheitszustand repräsentieren, ausgewertet. Die Referenzpunkte können durch Glucosetoleranztests an Probanden ermittelt werden, deren jeweiliger Gesundheitszustand durch eine unabhängige Diagnose genau bekannt ist. Für Referenzpunkte können beispielsweise die Gesundheitszustände eines völlig gesunden Patienten (H-NG), eines Patienten mit insulinpflichtiger Typ-II-Diabetes (DT2), eines normoglykämischen Musterpatienten mit metabolischem Syndrom (MS-NG), eines Musterpatienten mit metabolischem Syndrom und gestörter Glucosetoleranz (MS-IGT), einem Musterpatienten mit metabolischem Syndrom und gestörter Nüchternglucose (MS-IFG) und eines Musterpatienten, bei dem die Erhöhung der Nüchternglucose mit gestörter Glucosetoleranz kombiniert ist (MS-CGI), verwendet werden.

Um den Einfluss individueller Schwankungen und Besonderheiten zu minimieren, kann ein Referenzpunkt auch jeweils durch Mittelung der Ergebnisse von oralen Glucosetoleranztests mehrer Probanden mit gleichem Gesundheitszustand ermittelt werden.

Die Lage des Punkts, der den Datensatz eines Individuums mit unbekanntem Gesundheitszustand repräsentiert, kann beispielsweise ausgewertet werden, indem jeweils der Abstand zu den verschiedenen Referenzpunkten berechnet wird. Der unbekannte Gesundheitszustand kann dann dem Referenzpunkt zugeordnet werden, zu dem der kleinste Abstand ermittelt wird. Die Größe dieses Abstands ist dabei ein Maß für die Zuverlässigkeit dieser Zuordnung. Der Abstand kann also als Parameter verwendet werden, der den Zustand des Glucosestoffwechsels des Patienten angibt. Bevorzugt wird die Lage des Punkts, der den Datensatz eines Individuums mit unbekanntem Gesundheitszustand repräsentiert, aber ausgewertet, indem der Punkt auf eine Normtrajektorie projiziert wird, welche in dem Vektorraum einen Krankheitsverlauf von einem gesunden Normpatienten über einen der prädiabetischen Zustände bis zu einer schweren Diabeteserkrankung wiedergibt. Eine solche Normtrajektorie enthält zumindest einen Teil der vorstehend erwähnten Referenzpunkte und kann aus den Messdaten von oralen Glucosetoleranztests einer größeren Anzahl von Probanden ermittelt werden, deren jeweiliger Gesundheitszustand durch eine unabhängige Diagnose genau bekannt ist. Für jeden Datensatz eines Probanden kann dann ein Punkt in dem Vektorraum berechnet werden. Diese Punkte liegen - von Messfehlern und natürlichen Schwankungen abgesehen - auf einer Linie, die einen Datenpunkt eines gesunden Probanden mit dem Datenpunkt eines schwer diabeteskranken Probanden verbindet. Zwischen diesen Anfangs- und Endpunkten der Trajektorie liegen Punkte von Probanden verschiedener Krankheitsstadien.

In diesem Zusammenhang ist bedeutsam, dass die Trajektorie zwar zur erfindungsgemäßen Auswertung eines Satzes von Messdaten eines oralen Glucosetoleranztests benötigt wird, jedoch nicht für jede Auswertung neu berechnet werden muss. Es genügt, eine solche Normtrajektorie ein einziges Mal durch Auswertung einer großen Anzahl von Messdaten einer Vielzahl von Probanden unterschiedlichen und genau bekannten Gesundheitszustands zu ermitteln. Wenn das erfindungsgemäße Verfahren mit einem Computerprogramm durchgeführt wird, kann die Trajektorie deshalb vom Hersteller des Programms fest vorgegeben werden. Ein Benutzer des Programms hat dann keinen Einfluss auf den Verlauf der Trajektorie oder deren Werte.

Idealerweise legt der den auszuwertenden Satz von Messdaten präsentierende Punkt direkt auf der Normtrajektorie. Wegen unvermeidlichen Messungenauigkeiten und natürlichen Schwankungen ist aber in der Praxis damit zu rechnen, dass der den Messdatensatz repräsentierende Punkt einen mehr oder weniger großen Abstand von der Normtrajektorie hat. In diesem Fall wird in einem weiteren Verfahrensschritt der Punkt der Normtrajektorie ermittelt, der am nächsten an dem den auszuwertenden Datensatz repräsentierenden Punkt liegt. Dieser Punkt der Normtrajektorie wird also durch Projektion des den Messdatensatz repräsentierenden Punkts auf die Normtrajektorie ermittelt.

Dieser Punkt der Normtrajektorie unterteilt diese in zwei Abschnitte, nämlich einen Anfangsabschnitt vom Anfang der Normtrajektorie bis zu diesem Punkt und in einen Endabschnitt, von diesem Punkt der Trajektorie bis zum Ende der Trajektorie.

Die Längenverhältnisse dieser beiden Abschnitte der Trajektorie enthalten die Information, wie weit der Gesundheitszustand des Patienten von einem vollständig gesunden Zustand ohne Diabetesrisiko und von dem Vollbild einer schweren Diabeteserkrankung entfernt ist. Aus der Länge eines Trajektorienabschnitts vom Anfang der Trajektorie bis zu dem Punkt der Trajektorie, auf welchen der den Datensatz repräsentierende Punkt projiziert wurde, kann deshalb ein Parameter ermittelt werden, der der das Ausmaß einer Störung des Glucosestoffwechsels quantifiziert. Der Parameter kann somit das Stadium oder Risiko einer Diabeteserkrankung angeben.

Der Parameter kann insbesondere als das Verhältnis der Länge des Anfangsabschnitts der Trajektorie zur Gesamtlänge der Trajektorie angegeben werden. Bei einem völlig gesunden Patienten liegt der Anfangspunkt der Normtrajektorie dem Punkt, der den auszuwertenden Satz von Messdaten repräsentiert, am nächsten, so dass der Anfangsabschnitt die Länge Null hat. Bei einem Patienten mit vollständig manifestierter Diabeteserkrankung liegt dagegen das Ende der Trajektorie dem Punkt, welcher den auszuwertenden Satz von Messdaten repräsentiert, am nächsten, so dass der Anfangsabschnitt der Trajektorie dividiert durch die Gesamtlänge der Trajektorie den Wert eins ergibt.

In diesem Zusammenhang ist bedeutsam, dass in dem betrachteten Vektorraum mehrere Normtrajektorien verlaufen können, die jeweils unterschiedliche Krankheitsverläufe von einem gesunden Normpatienten über einen prädiabetischen Zustand bis zu einer insulinpflichtigen Diabeteserkrankung angeben. Eine Diabeteserkrankung kann sich nämlich auf unterschiedliche Weise entwickeln und möglicher Weise auch auf unterschiedlichen Ursachen oder Schädigungen beruhen. Beispielsweise kann eine Diabeteserkrankung damit beginnen, dass die Anzahl Insulin produzierender Zellen in der Bauchspeicheldrüse abnimmt und deshalb die körpereigene Insulinproduktion zum Erliegen kommt. Eine Diabeteserkrankung kann aber auch damit beginnen, dass Muskelzellen Glucose zunehmend schlechter aufnehmen (Insulinresitenz), so dass erhöhte Insulinmengen oder eine Behandlung durch Insulinsensitizer benötigt werden. Sowohl die Therapiemöglichkeiten als auch die Krankheitsstadien, die auf dem Weg zum Endstadium einer Diabeteserkrankung durchlaufen werden, unterscheiden sich in beiden Fällen. Für jeden dieser Fälle kann deshalb in dem Vektorraum eine eigene Normtrajektorie betrachtet werde. Anfangs- und Endpunkte dieser Trajektorien stimmen überein, dazwischen haben die Trajektorien aber unterschiedliche Verläufe.

Falls in dem Vektorraum mehrere Normtrajektorien definiert sind, wird der den Datensatz charakterisierenden Punkt auf jene Normtrajektorie projiziert, zu der er den kleinsten Abstand hat. Zusätzlich kann der den Datensatz charakterisierenden Punkt auch auf eine zweite Normtrajektorie projiziert werden, zu der er den zweitkleinsten Abstand hat. Auf diese Weise kann mit der zweiten Normtrajektorie aus der Länge eines Trajektorienabschnitts von dem Anfang der Trajektorie bis zu dem Punkt der Trajektorie, auf welchen der den Datensatz repräsentierende Punkt projiziert wurde, ein zweiter Parameter ermittelt werden, der den Zustand des Glucosestoffwechsels angibt. Eine wertevolle Information in diesem Zusammenhang kann aus dem Abstand des den Datensatz repräsentierenden Punkts zu der ersten Normtrajektorie und dem Abstand zu der zweiten Normtrajektorie gewonnen werden, beispielsweise aus dem Verhältnis der beiden Abstände. Die Abstände gegeben nämlich an, mit welcher Zuverlässigkeit eine Zuordnung zu einer Normtrajektorie erfolgt ist und folglich ein weiterer Krankheitsverlauf erwartet werden kann. Fälle, in denen eine Zuordnung zu einer Normtrajektorie nicht eindeutig erfolgen kann, können insbesondere bei einem Prädiabetiker auf mehrere gleichzeitig auftretende Schädigungsmechanismen des Glucosestoffwechsels hindeuten.

Bei dem erfindungsgemäß betrachteten Vektorraum können alle Koordinatenachsen von Ähnlichkeitsmaßen gebildet werden. In diesem Fall können alle Koordinaten eines Punktes, der einen Datensatz eines oralen Glucosetoleranztests repräsentiert, als Werte von Ähnlichkeitsmaßen angegeben werden. Der betrachtete Vektorraum kann aber auch eine oder mehrere zusätzliche Dimensionen aufweisen, d. h. weitere Koordinatenachsen haben, zu denen Koordinatenwerte unabhängig von den Ähnlichkeitsmaßen berechnet werden. Beispielsweise kann eine weitere Koordinatenachse des Vektorraums von einer Norm eines Vektors gebildet werden, der aus der Folge von Messwerten der Glucosekonzentration oder der Analytkonzentration gebildeten ist. Der den Datensatz in dem Vektorraum repräsentierende Punkt hat dann als eine weitere Koordinate den Wert der betreffenden Norm. Die Norm kann in der üblichen Weise als euklidische Vektornorm berechnet werden, d. h. als Wurzel aus der Summe der Quadrate der einzelnen Vektorkomponenten.

Als Dimensionen des Vektorraums können auch biometrische oder genetische Variablen ergänzt werden. Beispiele für biometrische Variablen sind Body-Mass-Index, Körperfettanteil und Waist-to-Hip-Ratio. Ferner können auch Parameter wie Blutdruck oder Puls als Koordinaten verwendet werden. Geeignet sind auch Konzentrationen von Metaboliten, die auf der Zeitskala eines oralen Glucosetoleranztests nicht oder nur wenig veränderlich sind, wie Cholesterin, Cholesterinfraktionen (LDL und HDL), HbA1c oder Nierenfunktionsparameter.

Ein erfindungsgemäßes Verfahren lässt sich nur mit einem Datenverarbeitungsgerät, d.h. einem Computer effizient durchführen. Die vorliegende Erfindung betrifft deshalb auch ein Computerprogramm zur Durchführung eines erfindungsgemäßen Verfahrens. Ein erfindungsgemäßes Computerprogrammprodukt kann in den Speicher eines Computers geladen werden und führt die Schritte eines erfindungsgemäßen Verfahrens aus, wenn das Computerprogrammprodukt auf einem Computer läuft. Die Erfindung betrifft ferner auch ein maschinenlesbares Speichermedium, auf dem ein solches Computerprogrammprodukt gespeichert ist, also ein Programm, das ein erfindungsgemäßes Verfahren durchführt, wenn es auf einem Computer läuft.

Weitere Einzelheiten und Vorteile werden an einem Ausführungsbeispiel unter Bezugnahme auf die beigefügten Zeichnungen erläutert. Es zeigen:

Fig. 1    ein Beispiel für den Verlauf der Glucosekonzentration bei einem oralen Glucosetoleranztest für verschiedene Patientengruppen;

Fig. 2    ein Beispiel für den Verlauf der C-Peptid-Konzentration bei einem oralen Glucosetoleranztests für verschiedene Patientengruppen;

Fig. 3    ein Beispiel für den Proinsulinverlauf bei einem Glucosetoleranztests für verschiedene Patientengruppen; und

Fig. 4    Datenpunkte, die Ergebnisse von oralen Glucosetoleranztests für drei verschiedene Patientengruppen repräsentierend, in einem dreidimensionalen Vektorraum;

Fig. 5    eine weitere Ansicht zu Figur 4; und

Fig. 6    ein Beispiel einer Normtrajektorie mit Datenpunkten von oralen Glucosetoleranztests verschiedener Patientengruppen.

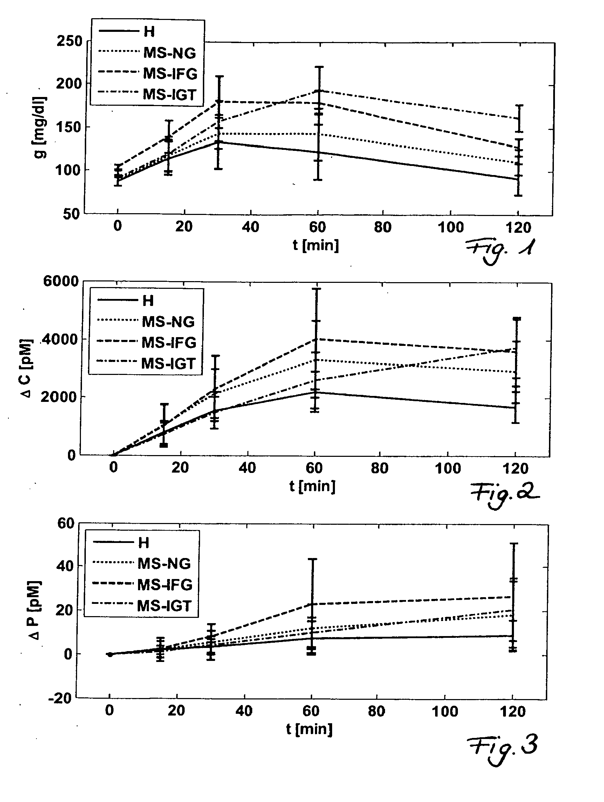

Figur 1 zeigt für verschiedene Patientengruppen den mittleren Verlauf der Glucosekonzentration g in mg pro dl über der Zeit t während eines Glucosetoleranztests über einen Zeitraum von 120 min. Bei den einzelnen Patientengruppen handelt es sich um völlig gesunde Menschen (H-(NG), normoglykämischen Patienten mit metabolischem Syndrom (MS-NG), Patienten mit metabolischem Syndrom und gestörter Glucosetoleranz (MS-IGT) und Patienten mit metabolischem Syndrom und gestörter Nüchternglucose (MS-IFG). Metabolisches Syndrom gilt als Risikofaktor für Typ-II Diabetes; eine Untersuchung speziell dieser Risikogruppe mit oralem Glucosetoleranztest ist daher sinnvoll. Daneben ist das erfindungsgemäße Verfahren aber auch für andere Risikogruppen - Risiko in Bezug auf Typ-II Diabetes - sinnvoll anwendbar, z.B. Übergewichtige oder Individuen mit genetischer Prädisposition.

Für die Patientengruppen von Figur 1 zeigt Figur 2 den durchschnittlichen Verlauf der C-Peptid-Konzentration während des oralen Glucosekonzentrationstests. In Figur 2 ist dabei aber nicht die absolute C-Peptid-Konzentration aufgetragen, sondern deren Änderung ∆C in Bezug auf einen Anfangswert. In entsprechender Weise zeigt Figur 3 die Änderung der Proinsulinkonzentration ∆P in Bezug auf einen Anfangswert während des Glucosetoleranztests.

Bei dem in den Figuren 1 bis 3 dargestellten Beispiel wurden Analytkonzentrationen jeweils zu Beginn des Glucosetoleranztest (t = 0), nach 15 min, nach 30 min, nach 60 min und nach 120 min gemessen. Messwerte der Glucosekonzentration werden im Folgenden mit $g_n$ bezeichnet, wobei n in Minuten die Zeit angibt, für welche der Konzentrationswert gemessen wurde. In entsprechender Weise werden Messwerte der C-Peptid-Konzentration, genauer gesagt der Änderung in Bezug auf einen Anfangswert, mit $C_n$ und Messwerte der Proinsulinkonzentration, genauer gesagt der Änderung in Bezug auf einen Anfangswert, mit $p_n$ bezeichnet.

Aus den Messwerten einer Analytkonzentration kann ein Vektor gebildet werden, indem jeder gemessene Konzentrationswert als eine Vektorkomponente verwendet wird. Aus den Messwerten der Glucosekonzentration kann im vorliegenden Fall also ein Vektor $\underline{g}$ = ($g_0$, $g_{15}$, $g_{30}$, $g_{60}$, $g_{120}$) gebildet werden. In entsprechender Weise können beispielsweise aus den Messwerten der C-Peptid-Konzentration oder der Proinsulinkonzentration Vektoren $\underline{c}$, $\underline{p}$ gebildet werden, welche die betreffende Folge von Messwerten repräsentieren.

Für die weitere Auswertung ist es vorteilhaft, die so gebildeten Vektoren zu normieren. Als Norm ist insbesondere die gewöhnliche quadratische Definition, d. h. die euklidische Vektornorm geeignet, also dann $\| \underline{g} \| = (\underline{g} \cdot \underline{g})^{1/2}$

Zur weiteren Untersuchung der Folgen von Messwerten bzw. der daraus gebildeten Vektoren legt man Musterverläufe der Glucosekonzentration und der anderen Analytkonzentrationen fest. Zu diesen Musterverläufen, die einen Abschnitt des bei einem gegeben Gesundheitszustand während eines oralen Glucosetoleranztests erwarteten zeitlichen Verlaufs der Analytkonzentration repräsentieren können, werden dann jeweils Vektoren gebildet.

Als Vektoren zu Musterverläufen werden bei dem vorliegenden Ausführungsbeispiel die folgenden Vektoren verwendet, wobei jeweils M die Dimension des Vektorraums ist:

- Der Vektor der normierten Raumdiagonalen $\underline{N}_M = (1/\sqrt{M}, 1/\sqrt{M}, \ldots 1/\sqrt{M})$. Enthält der Vektor also fünf Komponenten, wie dies bei einem Verlauf gemäß der Figuren 1 bis 3 der Fall ist, ergibt sich ein Vektor

$$\underline{N} = (1, 1, 1, 1, 1)/\sqrt{5}.$$

- Ein Vektor $\underline{L}_M = (1, 2, 3, ..., M)/\|\underline{L}_M\|$. also ein linear ansteigender Musterverlauf. Für das Beispiel der Figuren 1 bis 3 ist $\underline{L} = (1, 2, 3, 4, 5)/\sqrt{55}$.

- Ein dreieckiger (M ungerade) oder trapezoidaler (M gerade) Musterverlauf mit dem Vektor $\underline{D}_M = (1, 2, ..., M/2, M/2-1, ..., 1)/\|\underline{D}_M\|$. wobei M/2 für ungerade M aufzurunden ist. Für den Fall der Figuren 1 bis 3 ist

$$\underline{D} = (1, 2, 3, 2, 1)/\sqrt{19}.$$

In einem weiteren Verfahrensschritt wird ein Ähnlichkeitsmaß berechnet, welches die Ähnlichkeit zwischen dem Konzentrationsverlauf und dem jeweiligen Musterverlauf angibt. Das Ähnlichkeitsmaß kann beispielsweise durch Bildung des Skalarprodukts zwischen den betreffenden Vektoren berechnet werden. Dabei kann das Skalarprodukt selbst als Ähnlichkeitsmaß verwendet werden oder aus dem Skalarprodukt der Winkel zwischen den beiden Vektoren berechnet werden.

Als Ähnlichkeitsmaß für den Verlauf der Glucosekonzentration können beispielsweise die folgenden Winkel verwendet werden:

$$\alpha_g = \arccos(\underline{N} \cdot \underline{g}), \quad \beta_g = \arccos(\underline{L} \cdot \underline{g}), \quad \gamma_g = \arccos(\underline{D} \cdot \underline{g}).$$

Entsprechend können als Ähnlichkeitsmaß für den Verlauf der C-Peptid- bzw. der Proinsulinkonzentration die Winkel

$$\alpha_c = \arccos(\underline{N} \cdot \underline{c}), \quad \beta_c = \arccos(\underline{L} \cdot \underline{c}), \quad \gamma_c = \arccos(\underline{D} \cdot \underline{c}) \text{ bzw.}$$

$$\alpha_p = \arccos(\underline{N} \cdot \underline{p}), \quad \beta_p = \arccos(\underline{L} \cdot \underline{p}), \quad \gamma_p = \arccos(\underline{D} \cdot \underline{p})$$

verwendet werden.

Jeder Datensatz eine oralen Glucosetoleranztests kann durch derartige Ähnlichkeitsmaße beschrieben werden. Ergänzend zur Charakterisierung eines Satzes von Messdaten eines Glucosetoleranztests auch eine Norm der einzelnen Vektoren, welche die Konzentrationsverläufe repräsentieren, verwendet werden. Also beispielsweise die euklidische Norm der Vektoren $\mathbf{g}$, $\mathbf{c}$, $\mathbf{p}$. Für eine Satz von Messdaten erhält man so also eine Satz von Variablen, beispielsweise die Variablen $\alpha_g$, $\beta_g$, $\gamma_g$, $\alpha_c$, $\beta_c$, $\gamma_c$, $\alpha_p$, $\beta_p$, $\gamma_p$, $\|\mathbf{g}\|$, $\|\mathbf{c}\|$, $\|\mathbf{p}\|$.

Hierbei ist anzumerken, dass auch eine größere oder kleinere Anzahl von Musterverläufen und insbesondere auch andere Musterverläufe verwendet werden können. Insbesondere können für die einzelnen Analytkonzentrationen auch jeweils unterschiedliche Musterverläufe verwendet werden. Ein Satz von Messdaten kann deshalb auch durch einen anderen Variablensatz mit einer größeren oder kleineren Anzahl von Variablen charakterisiert werden.

Die Werte der einzelnen Variablen des aus den Messdaten berechneten Variablensatzes, also beispielsweise die Werte der Variablen $\alpha_g$, $\beta_g$, $\gamma_g$, $\alpha_c$, $\beta_c$, $\gamma_c$, $\alpha_p$, $\beta_p$, $Y_p$, $\|\mathbf{g}\|$, $\|\mathbf{c}\|$, $\|\mathbf{p}\|$, können als Koordinaten in einem Vektorraum verwendet werden. Auf diese Weise lässt sich jeder Satz von Messdaten eines Glucosetoleranztests durch einen Punkt in einem Vektorraum repräsentieren. Die Koordinatenachsen des Vektorraumes sind dann jeweils durch eine der Variablen gegeben, wobei der Wert dieser Variablen die betreffende Koordinate angibt.

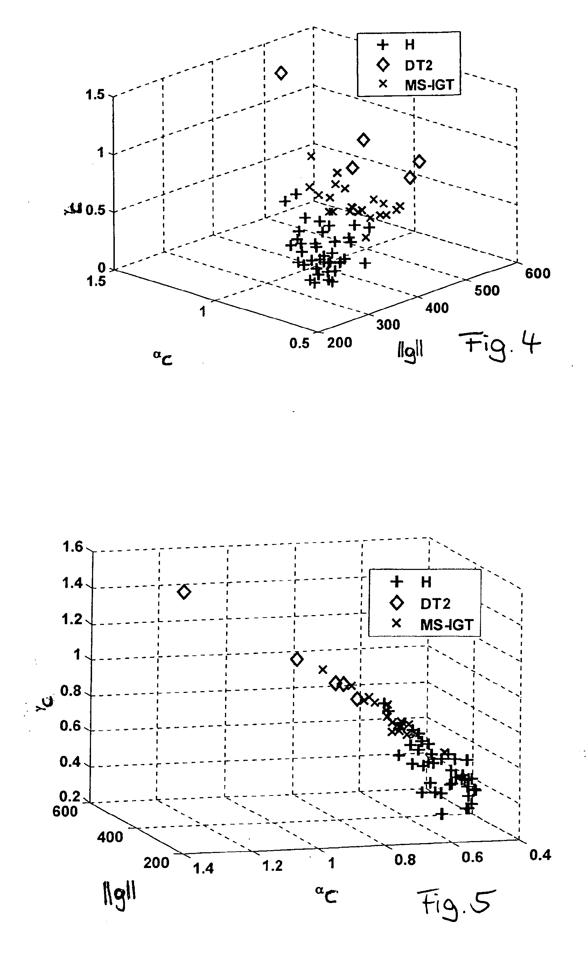

Die Figuren 4 und 5 zeigen schematisch in verschieden Blickwinkeln ein vereinfachtes Beispiel eines solchen Vektorraums, in dem Punkte eingetragen sind, die jeweils einen Satz von Messdaten eines oralen Glucosetoleranztests repräsentieren. Da nur drei Dimensionen graphisch darstellbar sind, wurden für Figuren 4 und 5 von den vorstehend genannten Variablen zur Veranschaulichung die Variablen $\|\mathbf{g}\|$, $\alpha_c$ und $\gamma_c$ ausgewählt. Bei einer praktischen Durchführung des Verfahrens wird aber ein Vektorraum mit einer höheren Dimension verwendet, also eine größere Anzahl von Variablen verwendet.

Bereits an dem vereinfachten Beispiel der Figuren 4 und 5 lässt sich aber erkennen, dass die Datenpunkte verschiedener Patientengruppen in dem Vektorraum deutlich getrennt sind. Datenpunkte von gesunden Menschen (H) sind durch + dargestellt, Datenpunkte von Patienten mit metabolischem Syndrom und gestörter Glucosetoleranz (MS-IGT) durch x und Patienten mit Typ-II-Diabetes (DT2) durch 0. In der Darstellung von Figur 5 lässt sich erkennen, dass die Datenpunkte näherungsweise in einer Ebene liegen und somit in Figur 5 eine Linie bilden. Im linken, oberen Abschnitt dieser Linie finden sich Datenpunkte von Patienten mit Typ-II-Diabetes (DT2). Im rechten, unteren Abschnitt liegen Datenpunkte von gesunden Proben (H). Entlang der Linie finden sich also von rechts nach links zunächst Datenpunkte vom Typ H, dann in zunehmendem Maß auch Datenpunkte vom Typ MS-IGT und schließlich Datenpunkte vom Typ DT2. Bereits in einem vereinfachten Vektorraum lässt sich also eine Linie erkennen, die angibt, wie sich bei fortschreitender Krankheit die aus einem oralen Glucosetoleranztest gewonnenen Daten ändern.

Indem Werte der einzelnen Variablen des aus den Messdaten berechneten Variablensatzes, also beispielsweise die Werte der Variablen $\alpha_g$, $\beta_g$, $\gamma_g$, $\alpha_c$, $\beta_c$, $\gamma_c$, $\alpha_p$, $\beta_p$, $\gamma_p$, $\|\mathbf{g}\|$, $\|\mathbf{c}\|$, $\|\mathbf{p}\|$, als Koordinaten in einem Vektorraum verwendet werden, wird deshalb ein Vektorraum definiert, in dem eine Trajektorie verläuft, die einen typischen Krankheitsverlauf mit zunehmender Störung des Glucosemetabolismus angibt. Die Trajektorie beginnt also mit einem Punkt, der für einen völlig gesunden Zustand zu erwarten ist, und verläuft über Punkte, die prädiabetische Zustände oder eine beginnende Diabeteserkrankung repräsentieren, bis hin zu einem Punkt, dessen Koordinaten als Variablenwerte bei einem Patienten mit einer insulinpflichtigen Diabeteserkrankung auftreten.

Diese Trajektorie kann als Normtrajektorie bezeichnet werden, da sie den normalen Krankheitsverlauf beschreibt. Eine Normtrajektorie kann durch Auswertung der Daten einer größeren Anzahl oraler Glucosetoleranztests von Probanden mit bekanntem Gesundheitszustand gewonnen werden.

Indem man für jedes Stadium einer Diabeteserkrankung Daten von oralen Glucosetoleranztests auswertet, erhält man aus jedem Satz von Messdaten einen Punkt in dem Vektorraum. Bei identischem Gesundheitszustand sollten diese Punkte zusammenfallen. Tatsächlich ist aber nicht davon auszugehen, dass zwei Patienten exakt denselben Gesundheitszustand haben, so dass mit einer Streuung der Punkte zu rechnen ist. Trotz einer solchen Streuung lässt sich aus einer ausreichend großen Anzahl von Datenpunkten eine Normtrajektorie berechnen, beispielsweise durch Mittelwertbildung. Bevorzugt wird für Datensätze, die an Patienten mit übereinstimmendem Gesundheitszustand gemessen wurden, über die auftretenden Werte einer Variablen gemittelt, gewissermaßen der Schwerpunkt einer Punktwolke ermittelt. Es ist aber auch möglich, schon in einem früheren Stadium der Datenauswertung einen Mittelwert zu bilden, beispielsweise kann bereits über die einzelnen Konzentrationsverläufe gemittelt werden, um einen typischen Verlauf der Konzentration von Glucose oder einem sonstigen Analyten für den betreffenden Gesundheitszustand bei einem Glucosetoleranztest zu ermitteln.

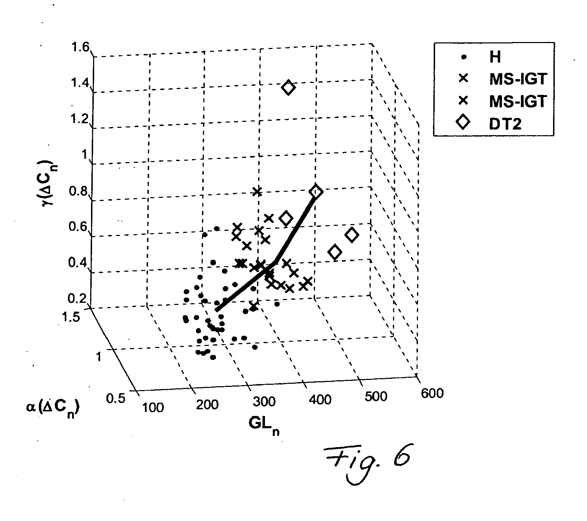

Figur 6 zeigt beispielhaft eine Normtrajektorie mit Datenpunkten von oralen Glucosetoleranztests verschiedener Patientengruppen, nämlich gesunden Patienten H (·), Patienten mit metabolischem Syndrom und gestörter Glucosetoleranz MS-IGT (x) und insulinpflichtigen Typ-II Diabetikern DT2 (0).

Um aus einem Satz von Messdaten eines oralen Glucosetoleranztests den Gesundheitszustand des Patienten zu bestimmen, wird ermittelt, welcher Punkt der Normtrajektorie den geringsten Abstand zu dem Punkt, der den Satz von Messdaten des Glucosetoleranztests repräsentiert, hat. Der die Messdaten eines Glucosetoleranztests repräsentierende Punkt wird also auf die Normtrajektorie projiziert. Der auf diese Weise ermittelte Punkt der Normtrajektorie unterteilt diese in zwei Abschnitte, nämlich einen Anfangsabschnitt und einen Endabschnitt. Aus der Länge des Trajektorienabschnitts, der sich vom Anfang der Trajektorie bis zu dem Punkt der Trajektorie, auf welche der den Datensatz repräsentierende Punkt projiziert wurde, wird dann ein Parameter ermittelt, der das Ausmaß einer Störung des Glucosestoffwechsels quantifiziert und somit das Krankheitsstadium des Patienten angibt. Der Parameter kann beispielsweise als Verhältnis der Länge des Anfangsabschnitts zur Gesamtlänge der Trajektorie angegeben werden.

Das Verfahren ermöglicht insbesondere auch eine Differenzierung innerhalb eines gegebene Krankheits- bzw. Gesundheitszustands. Beim Typ-II Diabetes erkennt man bestimmte Phasen der Erkrankung: Behandlung mit Diät, Behandlung mit Diät plus oraler Medikation (z.B. Metformin), Behandlung mit oraler Medikation plus Ergänzungsinsulin sowie voll insulinpflichtiger Typ-II Diabetes. Diese Progredienz kann in einem Vektorraum durch eine Normtrajektorie beschrieben werden. Der Datenpunkt eines untersuchten Diabetikers kann einem Punkt der Normtrajektorie zugeordnet werden und das Krankheitsstadium anhand der Lage auf der Normtrajektorie abgelesen werden. Vorteilhaft kann so erkannt werden, wann es an der Zeit ist von einer Behandlungsmethode auf die nächste umzustellen.

**Patentansprüche**

1.    Verfahren zur Auswertung eines Satzes von Messdaten eines oralen Glucosetoleranztests, wobei der Satz von Messdaten eine Folge von Messdaten der Glucosekonzentration und zusätzlich wenigstens eine Folge von Messwerten einer weiteren Analytkonzentration enthält, **dadurch gekennzeichnet, dass** aus der Folge von Messwerten der Glucosekonzentration und jeweils einem von mehreren vorgegebenen Glucose-Musterverläufen jeweils ein Wert eines Ähnlichkeitsmaß berechnet wird, das die Ähnlichkeit zwischen dem zeitlichen Verlauf der Folge von Messwerten der Glucosekonzentration und dem betreffenden Glucose-Musterverlauf quantifiziert,
aus der Folge von Messwerten der weiteren Analytkonzentration und mehreren vorgegebenen Analyt-Musterverläufen jeweils ein Wert eines weiteren Ähnlichkeitsmaß berechnet wird, das die Ähnlichkeit zwischen dem Verlauf der Folge von Messwerten der weiteren Analytkonzentration und dem betreffenden Analyt-Musterverlauf quantifiziert,
der Datensatz in einem Vektorraum, der von den Ähnlichkeitsmaßen gebildete Koordinatenachsen aufweist, durch einen Punkt charakterisiert wird, dessen Koordinaten die berechneten Werte der Ähnlichkeitsmaße enthalten,
die Lage dieses Punkts in Bezug auf Referenzpunkte, die jeweils einen definierten Gesundheitszustand repräsentieren, ausgewertet wird, um einen Parameter zu berechnen, der den Zustand des Glucosestoffwechsels des Patienten angibt.

2.    Verfahren nach Anspruch 1, **dadurch gekennzeichnet, dass** die Lage des den Datensatz charakterisierenden Punktes in Bezug auf die Referenzpunkte ausgewertet wird, indem der Punkt auf eine Normtrajektorie projiziert wird, welche in dem Vektorraum einem Krankheitsverlauf von einem gesunden Normpatienten über einen prä-diabetischen Zustand bis zu einer Diabeteserkrankung folgt und zumindest einen Teil der Referenzpunkte enthält, aus der Länge eines Trajektorienabschnitts von dem Anfang der Trajektorie bis zu dem Punkt der Trajektorie, auf welchen der den Datensatz repräsentierende Punkt projiziert wurde, der Parameter ermittelt wird, der den Zustand des Glucosestoffwechsels angibt.

3.    Verfahren nach Anspruch 2, **dadurch gekennzeichnet, dass** in dem Vektorraum mehrere Normtrajektorien verlaufen, die jeweils unterschiedliche Krankheitsverläufe von einem gesunden Normpatienten über einen prä-diabetischen Zustand bis zu einer insulinpflichtigen Diabeteserkrankung angeben, wobei der den Datensatz charakterisierenden Punkt auf jene Normtrajektorie projiziert wird, zu der er den kleinsten Abstand hat.

4.    Verfahren nach Anspruch 3, **dadurch gekennzeichnet, dass** der den Datensatz charakterisierenden Punkt zusätzlich auch auf eine zweite Normtrajektorie projiziert wird, zu der er den zweitkleinsten Abstand hat.

5.    Verfahren nach einem der vorstehenden Ansprüche, **dadurch gekennzeichnet, dass** die Konzentrationsverläufe vor Berechnung der Ähnlichkeitsmaße normiert werden.

6.    Verfahren nach einem der vorstehenden Ansprüche, **dadurch gekennzeichnet, dass** die Ähnlichkeitsmaße als Skalarprodukt von Vektoren berechnet werden, wobei jeweils einer der Vektoren aus der betreffenden Folge von Messwerten und der andere Vektor aus dem betreffenden Musterverlauf bestimmt wird.

7.    Verfahren nach Anspruch 5, **dadurch gekennzeichnet, dass** die Ähnlichkeitsmaße jeweils als das Skalarprodukt zwischen normierten Vektoren berechnet werden.

8. Verfahren nach einem der vorstehenden Ansprüche, **dadurch gekennzeichnet, dass** als weitere Koordinate des Vektorraums eine Norm eines aus der Folge von Messwerten der Glucosekonzentration gebildeten Vektors verwendet wird.

9. Verfahren nach einem der vorstehenden Ansprüche, **dadurch gekennzeichnet, dass** als weitere Koordinate des Vektorraums eine Norm eines aus der Folge von Messwerten der weiteren Analytkonzentration gebildeten Vektors verwendet wird.

10. Verfahren nach einem der vorstehenden Ansprüche, **dadurch gekennzeichnet, dass** wenigstens eine Koordinatenachse des Vektorraums den Wert einer biometrischen oder genetischen Variablen, die unabhängig von einer Konzentrationsmessung gemessen wird, angibt.

11. Verfahren nach Anspruch 10, **dadurch gekennzeichnet, dass** die biometrische Variable der Body-Mass-Index, Körperfettanteil, Waist-to-Hip-Ratio, Blutdruck oder Puls ist.

12. Verfahren nach einem der vorstehenden Ansprüche, **dadurch gekennzeichnet, dass** die weitere Analytkonzentration die Konzentration eines Sekretionshormons ist.

13. Verfahren nach einem der vorstehenden Ansprüche, **dadurch gekennzeichnet, dass** wenigstens eine Koordinatenachse des Vektorraums die Konzentrationen eines Metaboliten, die auf der Zeitskala eines oralen Glucosetoleranztests nicht oder nur wenig veränderlich ist, angibt.

14. Computerprogrammprodukt, das Softwareabschnitte enthält, mit denen die Schritte eines Verfahrens nach einem der vorstehenden Ansprüche durchgeführt werden kann, wenn das Computerprogrammprodukt auf einem Computer läuft.

Fig. 1

Fig. 2

Fig. 3

Fig. 4

Fig. 5

*Fig. 6*

EUROPÄISCHES PATENTAMT
Europäisches Patentamt
European Patent Office
Office européen des brevets

**EUROPÄISCHER RECHERCHENBERICHT**

**Nummer der Anmeldung**

EP 11 00 0081

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | SCHRUEFER ET AL: "Signalverarbeitung", SIGNALVERARBEITUNG: NUMERISCHE VERARBEITUNG DIGITALER SIGNALE, XX, XX, 1. Januar 1990 (1990-01-01), Seite COMPLETE, XP002223960, * Seite 1 * * Seite 235 - Seite 253 * ----- | 1-14 | INV. G09B23/00 |
| A | US 2007/179771 A1 (KOUCHI YASUHIRO [JP] ET AL) 2. August 2007 (2007-08-02) * das ganze Dokument * ----- | 1-14 | |

**RECHERCHIERTE SACHGEBIETE (IPC)**

G09B

Der vorliegende Recherchenbericht wurde für alle Patentansprüche erstellt

| Recherchenort | Abschlußdatum der Recherche | Prüfer |
|---|---|---|
| München | 14. Mai 2012 | Kemény, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                                                       
& : Mitglied der gleichen Patentfamilie, übereinstimmendes Dokument

EPO FORM 1503 03.82 (P04C03)

**EP 2 474 962 A1**

**ANHANG ZUM EUROPÄISCHEN RECHERCHENBERICHT
ÜBER DIE EUROPÄISCHE PATENTANMELDUNG NR.**

EP 11 00 0081

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2012

| Im Recherchenbericht angeführtes Patentdokument | Datum der Veröffentlichung | Mitglied(er) der Patentfamilie | | Datum der Veröffentlichung |
|---|---|---|---|---|
| US 2007179771 A1 | 02-08-2007 | EP US | 1830333 A1 2007179771 A1 | 05-09-2007 02-08-2007 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1830333 A1